# EUROPEAN PATENT SPECIFICATION

(11) **EP 3 915 597 B1**
(45) Date of publication and mention of the grant of the patent: **10.07.2024**
(21) Application number: 21172990.0
(22) Date of filing: 10.05.2021
(51) Int. Cl.: A61L 2/10, A45C 15/00, A45F 3/00, B64D 13/00, F21L 13/00

(54) **PORTABLE SANITIZING SYSTEM HAVING A BACKPACK ASSEMBLY COUPLED TO A WAND ASSEMBLY**
TRAGBARES DESINFEKTIONSSYSTEM MIT EINER AN EINE STABANORDNUNG GEKOPPELTEN RUCKSACKANORDNUNG
SYSTÈME DE DÉSINFECTION PORTABLE COMPORTANT UN ENSEMBLE DE SAC À DOS COUPLÉ À UN ENSEMBLE DE TUBE

(30) Priority: 08.05.2020 US 202063021984 P; 10.09.2020 US 202017016466
(43) Date of publication of application: 01.12.2021
(73) Proprietor: The Boeing Company, Arlington, VA 22202 (US)
(72) Inventor: CHILDRESS, Jamie J, Chicago, 60606-2016 (US); KING, Teresa, Chicago, IL 60606-2016 (US)
(74) Representative: Boult Wade Tennant LLP

(56) References cited:
- WO-A1-2019/153014
- WO-A1-2019/170678
- KR-B1- 101 142 520
- US-A1- 2004 247 370
- US-A1- 2008 073 595
- US-B1- 6 630 105

## Description

### FIELD OF THE DISCLOSURE

Examples of the present disclosure generally relate to sanitizing systems, such as may be used to sanitize structures and areas within vehicles, such as commercial aircraft.

### BACKGROUND OF THE DISCLOSURE

Vehicles such as commercial aircraft are used to transport passengers between various locations. Systems are currently being developed to disinfect or otherwise sanitize surfaces within aircraft, for example, that use ultraviolet (UV) light. In order to sanitize a surface of a structure, a known UV light sterilization method emits a broad spectrum UVC light onto the structure.

However, certain UV light sanitizing systems are typically large, bulky, and often require fixed, stationary infrastructure.

WO 2019/153014 A1, in accordance with its abstract, states a unified airflow system for ultraviolet disinfection devices are disclosed. Included is a unified airflow assembly and a control unit. The unified airflow assembly provides a shared airflow passage between a UV source and an airflow accessory capable of extracting contaminants from a target surface using a suction airstream. The UV source may be fluidically disconnected from the airflow accessory. The unified airflow assembly may include at least one air restriction unit in the shared airflow passage for manipulating a suction airstream therein. The control unit may be configured to drive the at least one air restriction unit to restrict the suction airstream to only one of the UV source and the airflow accessory.

US 6630105 B1, in accordance with its abstract, states an apparatus and method for decontaminating chemical and biological agents using the reactive properties of both the single atomic oxygen and the hydroxyl radical for the decontamination of chemical and biological agents. The apparatus is self contained and portable and allows for the application of gas reactants directly at the required decontamination point. The system provides for the use of ultraviolet light of a specific spectral range to photolytically break down ozone into molecular oxygen and hydroxyl radicals where some of the molecular oxygen is in the first excited state. The excited molecular oxygen will combine with water vapor to produce two hydroxyl radicals.

WO 2019/170678 A1, in accordance with its abstract, states a portable unit with lance, incorporating a steam generator and a tank of sanitiser which can optimise the sanitising and disinfecting of surfaces by steam, and with an assembly of pipes and electrical supply cable and a human-machine interface , which authorises the supply of fluids and electrical power, either in-situ on any local network which has a supply of water and electricity and a centralised air blowing/sucking device, or on a module that can be carried by a user which incorporates autonomous means for managing fluids and electricity.

KR 101142520 B1, in accordance with a machine translation of its abstract, states a vacuum cleaner having a sterilization function using a ruler and a linear light emitting diode. UV light emitting diode in a vacuum cleaner that makes a vacuum shop and cleans it by sucking dust and other air together with the outside air equipped with a sterilization device made with a chip type, sterilizing together with cleaning the dust of the object to be cleaned, environmental problems, sterilization.

US 2004/247370 A1, in accordance with its abstract, states a mop assembly including a mop head and handle, and a supply of liquid connected to a valve assembly on the handle adjacent the mop head. The valve assembly includes a length of resiliently flexible tubing that has a portion extending transversely through the handle and first and second members on opposite sides of the length of tubing that are relatively movable between a closed position at which the first and second members pinch together opposite sides of the length of tubing to stop the flow of liquid through it, and an open position at which the first and second members are spaced apart to afford flow of liquid through it. Should the tubing become plugged, it can be replaced by disconnecting one end from a liquid supply length of tubing, withdrawing the tubing from between the first and second members, and then installing a new length of tubing. A storage cart for use with the mop assembly includes an elongate upwardly projecting main portion having walls providing a side surface, and an elongate tray portion that provides a receptacle adapted to receive the mop head. The tray portion is mounted on a bottom end of the main portion for relative movement between a closed position at which the side surface covers an open side of the receptacle to thereby afford retention of the mop head in the receptacle; and an open position with the tray portion disposed at about a right angle with respect to the main portion so that the mop head can be inserted into or removed from the receptacle and so that the bottom wall can be supported along a horizontal surface such as a floor surface with side walls of the receptacle projecting upwardly.

US 2008/073595 A1, in accordance with its abstract, states an ultra-violet cleaning and sterilization device that emits sufficient radiation to eradicate germs, bacteria, viruses and other pathogens and microorganisms with a replaceable and disposable outer fabric which cleans the surface and a ultra-violet emitting source sterilizing the surface with an on/off switch and a safety button which activates the ultra-violet emitting source by applying pressure to the button while wiping or cleaning a surface. US2006017025A1 discloses an ultraviolet gun comprising an airflow device to cool the light source.

### SUMMARY OF THE DISCLOSURE

A need exists for a system and a method for efficiently sterilizing surfaces within an internal cabin of a vehicle. Further, a need exists for a system and a method that allow for sterilization in confined and/or tight spaces. Additionally, a need exists for a system and a method that are able to quickly sterilize areas, such as within internal cabins of vehicles, establishments such as restaurants, entertainment venues (such as stadiums), and the like.

Described herein is a portable sanitizing system, comprising: a wand assembly including a sanitizing head having an ultraviolet (UV) lamp; and a backpack assembly coupled to the wand assembly, wherein the backpack assembly is removably securable to an individual through a harness; wherein the backpack assembly comprises an airflow device that is configured to generate airflow to cool the UV lamp, and to draw air from the sanitizing head of the wand assembly.

The ultraviolet (UV) light source may be a lamp (excimer lamp, mercury vapor, UV LED, and/or the like). A coupler, such as a hose, may couple the backpack assembly to the wand assembly. The coupler may include a hose and/or wiring. The wiring may be fitted within the hose. The hose may be used to move air around or within the wand assembly or the light source for coupling purposes or ozone mitigation.

The airflow device may be further configured to remove ozone from the sanitizing head.

The backpack assembly may include at least one air filter that is configured to filter air drawn from the sanitizing head of the wand assembly.

The backpack assembly may contain one or more batteries that provide power to the UV lamp. The UV assembly may be powered by a power source in addition to, or in lieu of, batteries. For example, the power source may be a 115V, 400Hz source or a 115V, 50/60Hz source.

The UV lamp may be configured to emit the UV light having a wavelength between 200 nm - 230 nm. The UV lamp may be configured to emit the UV having a wavelength of 222 nm.

The UV lamp may be configured to emit the UV light having a wavelength between 230 nm - 280 nm. The UV lamp may be configured to emit the UV light having a wavelength of 254 nm.

The wand assembly may further include a handle coupled to the sanitizing head. Further, the wand assembly can also include a coupler that moveably couples the handle to the sanitizing head. Further, the sanitizing head can be configured to one or both of linearly translate or swivel in relation to the handle.

The sanitizing head may include a shroud that retains the UV lamp. The shroud may include one or more openings that are configured to allow air to flow into the shroud.

A reflector may be secured to an underside of the shroud. The reflector may be configured to reflect a portion of the UV light that is emitted by the UV lamp.

The sanitizing head may further include a reflector, and a cover plate. The UV lamp may be secured within an interior chamber defined between a reflector and the cover plate.

Also described herein is a method of sanitizing a structure of a vehicle, such as a cockpit and/or a passenger section of an internal cabin of a commercial aircraft, using the portable sanitizing system described above, the method comprising: an individual wearing the portable sanitizing system while the walking through the vehicle as the UV lamp emits the UV light onto the structure.

### BRIEF DESCRIPTION OF THE DRAWINGS

Figure 1 illustrates a perspective view of a portable sanitizing system worn by an individual.
Figure 2 illustrates a perspective lateral top view of a wand assembly.
Figure 3 illustrates a perspective rear view of the wand assembly of Figure 2.
Figure 4 illustrates a perspective lateral view of the wand assembly of Figure 2.
Figure 5 illustrates a perspective view of the portable sanitizing system in a compact deployed position.
Figure 6 illustrates a perspective view of the portable sanitizing system having a sanitizing head in an extended position.
Figure 7 illustrates a perspective view of the portable sanitizing system having the sanitizing head in an extended position and a handle in an extended position.
Figure 8 illustrates a perspective view of the portable sanitizing system having the sanitizing head rotated in relation to the handle.
Figure 9 illustrates a perspective end view of a UV lamp and a reflector of the sanitizing head.
Figure 10 illustrates a perspective end view of a UV lamp and a reflector of the sanitizing head.
Figure 11 illustrates a perspective end view of a UV lamp and a reflector of the sanitizing head.
Figure 12 illustrates a perspective top view of the sanitizing head.
Figure 13 illustrates a perspective bottom view of the sanitizing head.
Figure 14 illustrates an axial cross-sectional view of the sanitizing head through line 14-14 of Figure 12.
Figure 15 illustrates a perspective end view of the UV lamp secured to a mounting bracket.
Figure 16 illustrates a perspective exploded view of a backpack assembly.
Figure 17 illustrates a perspective front view of a harness coupled to a backpack assembly.
Figure 18 illustrates an ultraviolet light spectrum.
Figure 19 illustrates a perspective front view of an aircraft.
Figure 20A illustrates a top plan view of an internal cabin of an aircraft.
Figure 20B illustrates a top plan view of an internal cabin of an aircraft.
Figure 21 illustrates a perspective interior view of an internal cabin of an aircraft.
Figure 22 illustrates a perspective internal view of a lavatory within an internal cabin of an aircraft.

### DETAILED DESCRIPTION OF THE DISCLOSURE

The foregoing summary, as well as the following detailed description of certain examples will be better understood when read in conjunction with the appended drawings. As used herein, an element or step recited in the singular and preceded by the word "a" or "an" should be understood as not necessarily excluding the plural of the elements or steps. Further, references to "one example" are not intended to be interpreted as excluding the existence of additional examples that also incorporate the recited features. Moreover, unless explicitly stated to the contrary, examples "comprising" or "having" an element or a plurality of elements having a particular condition can include additional elements not having that condition.

Certain examples of the present disclosure provide a portable sanitizing system for disinfecting surfaces, such as within an internal cabin of a vehicle. The portable sanitizing system includes a wand assembly and a backpack assembly. The wand assembly includes a housing, an ultraviolet (UV) lamp, a reflector, mounts to secure the UV lamp to the housing, an inlet to allow air to be drawn across the UV lamp, and an extension handle that is configured to extend a reach of the wand assembly. The backpack assembly includes a main body or housing, a power supply, one or more batteries (such as rechargeable batteries), a plug for recharging the backpack, an air blower, a carbon filter, an exhaust vent, and a harness to allow an individual to wear the portable sanitizing system.

The UV lamp is configured to emit UV light to disinfect a component, such as a surface of a structure within a vehicle. In at least one example, the UV lamp is configured to emit the UV light in the far UV spectrum. In at least one other example, the UV lamp is configured to emit the UV light in the UVC spectrum.

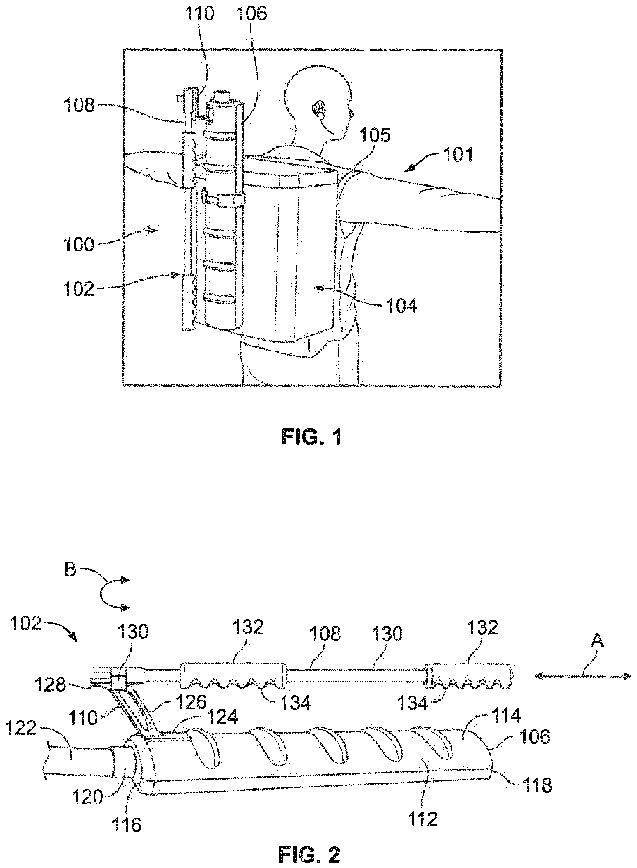

Figure 1 illustrates a perspective view of a portable sanitizing system 100 worn by an individual 101, according to an example of the present disclosure. The portable sanitizing system 100 includes a first assembly, such as a wand assembly 102, coupled to a second assembly, such as a backpack assembly 104 that is removably secured to the individual 101 through a harness 105. The wand assembly 102 includes a sanitizing head 106 coupled to a handle 108. In at least one example, the sanitizing head 106 is moveably coupled to the handle 108 through a coupler 110.

Optionally, the sanitizing head 106 is not moveably coupled to the handle 108. For example, the sanitizing head 106 can be fixed in relation to the handle 108. In at least one example, the sanitizing head 106 includes at least a portion that is integrally formed with and/or fixed to at least a portion of the handle 108.

As shown in Figure 1, the wand assembly 102 is in a stowed position. In the stowed position, the wand assembly 102 is removably secured to a portion of the backpack assembly 104, such as through one or more tracks, clips, latches, belts, ties, and/or the like.

Figure 2 illustrates a perspective lateral top view of the wand assembly 102, according to an example of the present disclosure. The sanitizing head 106 couples to the handle 108 through the coupler 110. The sanitizing head 106 includes a shroud 112 having an outer cover 114 that extends from a proximal end 116 to a distal end 118. As described herein, the shroud 112 contains a UV lamp. Optionally, the sanitizing head 106 coupled to the handle 108 without the coupler 110.

A port 120 extends from the proximal end 116. The port 120 couples to a hose 122, which, in turn, couples to the backpack assembly 104 (shown in Figure 1). The hose 122 contains electrical cords, cables, wiring, or the like that couples a power source or supply (such as one or more batteries) within the backpack assembly 104 (shown in Figure 1) to a UV lamp 140 within the shroud 112. Optionally, the electrical cords, cables, wiring, or the like may be outside of the hose 122. The hose 122 also contains an air delivery line, such as an air tube) that fluidly couples an internal chamber of the shroud 112 to an air blower, vacuum generator, air filters, and/or the like within the backpack assembly 104.

The coupler 110 is secured to the outer cover 114 of the shroud 112, such as proximate to the proximal end 116. The coupler 110 may include a securing beam 124 secured to the outer cover 114, such as through one or more fasteners, adhesives, and/or the like. An extension beam 126 outwardly extends from the securing beam 124, thereby spacing the handle 108 from the shroud 112. A bearing assembly 128 extends from the extension beam 126 opposite from the securing beam 124. The bearing assembly 128 includes one or more bearings, tracks, and/or the like, which allow the handle 108 to linearly translate relative to the coupler 110 in the directions of arrows A, and/or pivot about a pivot axle 129 in the directions of arc B. Optionally, the securing beam 124 may include a bearing assembly that allows the sanitizing head 106 to translate in the directions of arrows A, and/or rotate (for example, swivel) in the directions of arc B in addition to, or in place of, the handle 108 being coupled to the bearing assembly 128 (for example, the handle 108 may be fixed to the coupler 110).

In at least one example, the handle 108 includes a rod 130, pole, beam, or the like, which may be longer than the shroud 112. Optionally, the rod 130 may be shorter than the shroud 112. One or more grips 132 are secured to the rod 130. The grips 132 are configured to be grasped and held by an individual 101. The grips 132 may include ergonomic tactile features 134.

Optionally, the wand assembly 102 may be sized and shaped differently than shown. For example, the handle 108 may be fixed in relation to the shroud 112. Further, the handle 108 may or may not be configured to move relative to itself and/or the shroud 112. For example, the handle 108 and the shroud 112 may be integrally molded and formed as a single unit. As a further example, the wand assembly 102 can be sized, shaped, and configured as shown in United States Design Patent Application 29/735,235, filed May 19, 2020, entitled "Ultraviolet Wand."

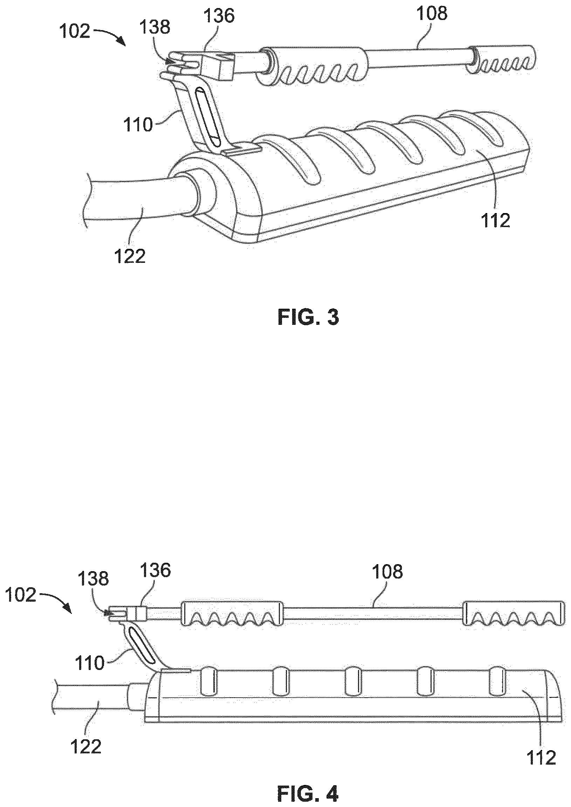

Figure 3 illustrates a perspective rear view of the wand assembly 102 of Figure 2. Figure 4 illustrates a perspective lateral view of the wand assembly 102 of Figure 2. Referring to Figures 3 and 4, the handle 108 may pivotally couple to the coupler 110 through a bearing 136 having a pivot axle 138 that pivotally couples the handle 108 to the coupler 110. The handle 108 may further be configured to linearly translate into and out of the bearing 136. For example, the handle 108 may be configured to telescope in and out. Optionally, or alternatively, in at least one example, the handle 108 may include a telescoping body that allows the handle 108 to outwardly extend and inwardly recede.

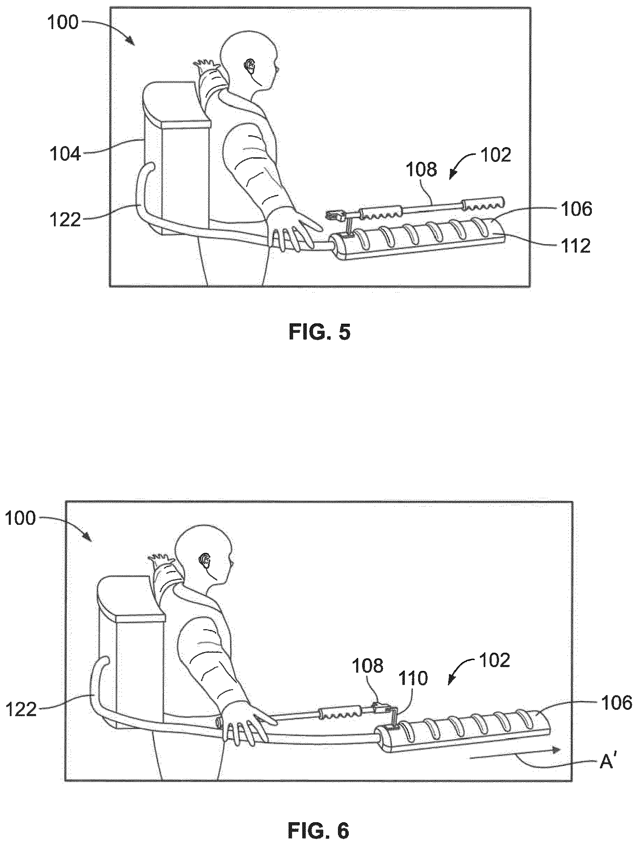

Figure 5 illustrates a perspective view of the portable sanitizing system 100 in a compact deployed position, according to an example of the present disclosure. The wand assembly 102 is removed from the backpack assembly 104 (as shown in Figure 1) into the compact deployed position, as shown in Figure 5. The hose 122 connects the wand assembly 102 to the backpack assembly 104. In the compact deployed position, the sanitizing head 106 is fully retracted in relation to the handle 108.

In at least one other example, backpack assembly 104 is configured to be opened and closed. An interior chamber 156 is defined within the backpack assembly 104. In at least one example, the wand assembly 102 is configured to be stored within the interior chamber 156 when not in use.

Figure 6 illustrates a perspective view of the portable sanitizing system 100 having the sanitizing head 106 in an extended position, according to an example of the present disclosure. In order to extend the sanitizing head 106 relative to the handle 108, the sanitizing head 106 is outwardly slid relative to the handle 108 in the direction of arrow A' (or the handle 108 is rearwardly slid relative to the sanitizing head 106). As noted, the sanitizing head 106 is able to linearly translate in the direction of arrow A' relative to the handle 108 via the coupler 110. The outward extension of the sanitizing head 106, as shown in Figure 6, allows for the portable sanitizing system 100 to easily reach distant areas. Alternatively, the sanitizing head 106 may not linearly translate relative to the handle 108.

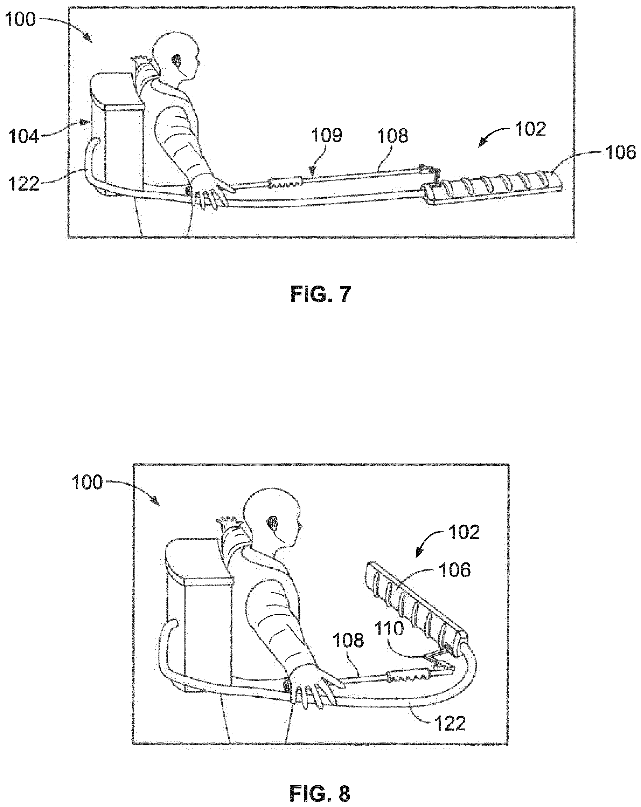

Figure 7 illustrates a perspective view of the portable sanitizing system 100 having the sanitizing head 106 in an extended position and the handle 108 in an extended position, according to an example of the present disclosure. To reach even further, the handle 108 may be configured to linearly translate, such as through a telescoping portion, to allow the sanitizing head 106 to reach further outwardly. Alternatively, the handle 108 may not be configured to extend and retract.

In at least one example, the handle 108 may include a lock 109. The lock 109 is configured to be selectively operated to secure the handle 108 into a desired extended (or retracted) position.

Figure 8 illustrates a perspective view of the portable sanitizing system 100 having the sanitizing head 106 rotated in relation to the handle 108, according to an example of the present disclosure. As noted, the sanitizing head 106 is configured to rotate relative to the handle 108 via the coupler 110. Rotating the sanitizing head 106 relative to the handle 108 allows the sanitizing head 106 to be moved to a desired position, and sweep or otherwise reach into areas that would otherwise be difficult to reach if the sanitizing head 106 was rigidly fixed to the handle 108. Alternatively, the sanitizing head 106 may not be rotatable relative to the handle 108.

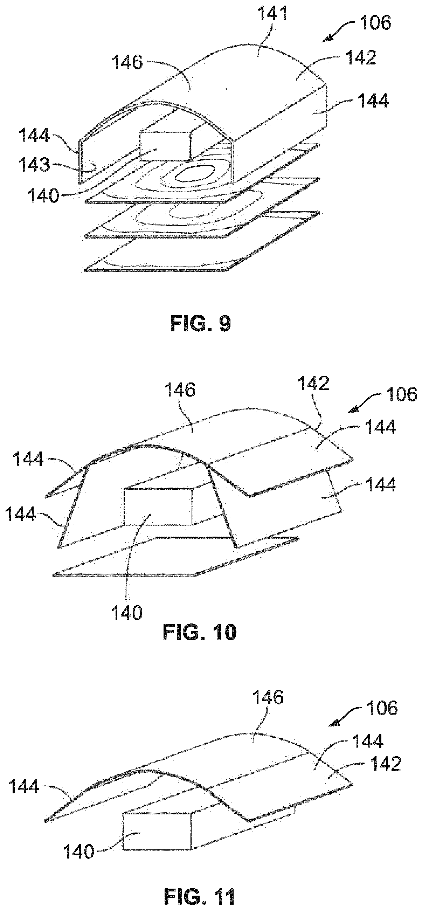

Figure 9 illustrates a perspective end view of a UV lamp 140 and a reflector 142 of the sanitizing head 106, according to an example of the present disclosure. The UV lamp 140 and the reflector 142 are secured within the shroud 112 (shown in Figure 2, for example) of the sanitizing head 106. In at least one example, the reflector 142 is secured to an underside 141 of the shroud 112, such as through one or more adhesives. In other examples, the reflector 142 is an integral part of the shroud 112. For example, the reflector 142 may be or otherwise provide the underside 141 of the shroud 112. The reflector 142 provides a reflective surface 143 (such as formed of Teflon, a mirrored surface, and/or the like) that is configured to outwardly reflect UV light emitted by the UV lamp 140. In at least one example, shroud 112 may be or include a shell formed of fiberglass, and the reflector 142 may be formed of Teflon that provides a 98% reflectivity.

The reflector 142 may extend along an entire length of the underside 141 of the shroud 112. Optionally, the reflector 142 may extend along less than an entire length of the underside 141 of the shroud 112.

The UV lamp 140 may extend along an entire length (or along substantially the entire length, such as between the ends 116 and 118). The UV lamp 140 is secured to the reflector 142 and/or the shroud 112 through one or more brackets, for example. The UV lamp 140 includes one or more UV light emitters, such as one more bulbs, light emitting elements (such as light emitting diodes), and/or the like. In at least one example, the UV lamp 140 is configured to emit UV light in the far UV spectrum, such as at a wavelength between 200 nm - 230 nm. In at least one example, the UV lamp 140 is configured to emit UV light having a wavelength of 222 nm. For example, the UV lamp 140 may be or include a 300 W bulb that is configured to emit UV light having a wavelength of 222 nm.

In at least one other example, the UV lamp 140 is configured to emit UV light in the UVC spectrum, such as at a wavelength between 230 - 280 nm. For example, the UV lamp 140 may be configured to emit UV light having a wavelength of 254 nm.

As shown, the reflector 142 includes flat, upright side walls 144 connected together through an upper curved wall 146. The upper curved wall 146 may be bowed outwardly away from the UV lamp 140. For example, the upper curved wall 146 may have a parabolic cross-section and/or profile.

It has been found that the straight, linear side walls 144 provide desired reflection and/or focusing of UV light emitted from the UV lamp 140 toward and onto a desired location. Alternatively, the side walls 144 may not be linear and flat.

Figure 10 illustrates a perspective end view of the UV lamp 140 and a reflector 142 of the sanitizing head, according to an example of the present disclosure. The reflector 142 shown in Figure 10 is similar to the reflector 142 shown in Figure 9, except that the side walls 144 may outwardly cant from the upper curved wall 146.

Figure 11 illustrates a perspective end view of the UV lamp 140 and the reflector 142 of the sanitizing head, according to an example of the present disclosure. In this example, the side walls 144 may be curved according to the curvature of the upper curved wall 146.

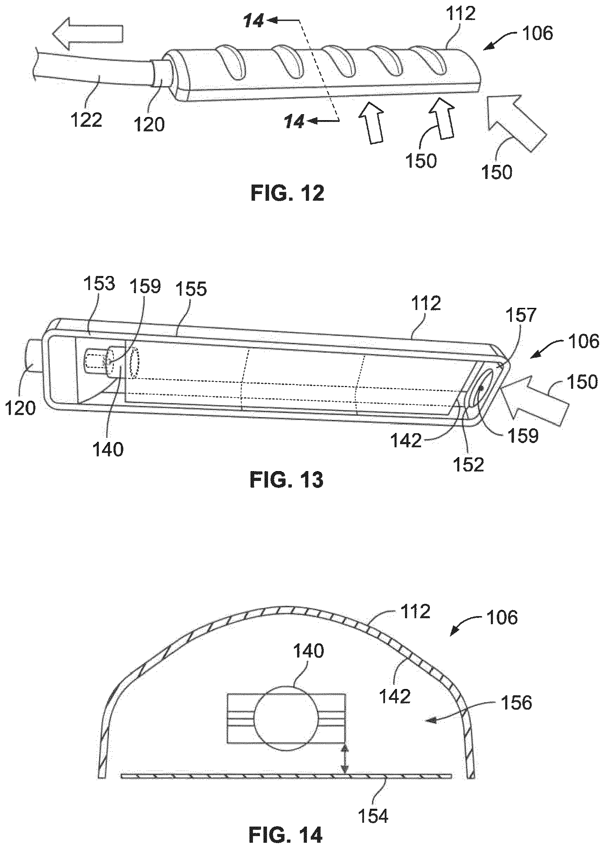

Figure 12 illustrates a perspective top view of the sanitizing head 106. Figure 13 illustrates a perspective bottom view of the sanitizing head 106. Figure 14 illustrates an axial cross-sectional view of the sanitizing head 106 through line 14-14 of Figure 12. Referring to Figures 12-14, air 150 is configured to be drawn into the sanitizing head 106 through one or more openings 152 (or simply an open chamber) of the shroud 112. The air 150 is drawn into the sanitizing head 106, such as via a vacuum generator within the backpack assembly 104 (shown in Figure 1). The air 150 is drawn into the shroud 112, and cools the UV lamp 140 as it passes over and around the UV lamp 140. The air 150 passes into the port 120 and into the hose 122, such as within an air tube within the hose 122. The air 150 not only cools the UV lamp 140, but also removes ozone, which may be generated by operation of the UV lamp 140, within the shroud 112. The air 150 may be drawn to an air filter, such as an activated carbon filter, within the backpack assembly 104.

In at least one example, the portable sanitizing system 100 may also include an alternative ozone mitigation system. As an example, the ozone mitigation system may be disposed in the shroud 112 or another portion of the system, and may include an inert gas bath, or a face inert gas system, such as in U.S. Patent No. 10,232,954.

Referring to Figure 13, in particular, a bumper 153 may be secured to an exposed lower circumferential edge 155 of the shroud 112. The bumper 153 may be formed of a resilient material, such as rubber, another elastomeric material, open or closed cell foam, and/or the like. The bumper 153 protects the sanitizing head 106 from damage in case the sanitizing head 106 inadvertently contacts a surface. The bumper 153 also protects the surface from damage.

The openings 152 may be spaced around the lower surface of the shroud 112 such that they do not provide a direct view of the UV lamp 140. For example, the openings 152 may be positioned underneath portions that are spaced apart from the UV lamp 140.

Referring to Figure 14, in particular, the sanitizing head 106 may include a cover plate 154 below the UV lamp 140. The cover plate 154 may be formed of glass, for example, and may be configured to filter UV light emitted by the UV lamp 140. The UV lamp 140 may be secured within an interior chamber 156 defined between the reflector 142 and the cover plate 154. In at least one example, the cover plate 154 is or otherwise includes a far UV band pass filter. For example, the cover plate 154 may be a 222 nm band pass filter that filters UV light emitted by the UV lamp 140 to a 222 nm wavelength. As such, UV light that is emitted from the sanitizing head 106 may be emitted at a wavelength of 222 nm. Optionally, the cover plate 154 may not be or otherwise include a far UV band pass filter. In at least one other example, the cover plate 154 is or otherwise includes a UVC band pass filter.

Referring to Figures 13 and 14, a rim 157 (such as a 0.020" (0.051 cm) thick Titanium rim) may connect the cover plate 154 to the shroud 112. The rim 157 may distribute impact loads therethrough and/or therearound.

In at least one example, ranging light emitting diodes (LEDs) 159 may be disposed proximate to ends of the UV lamp 140. The ranging LEDs 159 may be used to determine a desired range to a structure that is to be sanitized, for example. In at least one example, the ranging LEDs 159 may be disposed on or within the rim 157 and/or the cover plate 154.

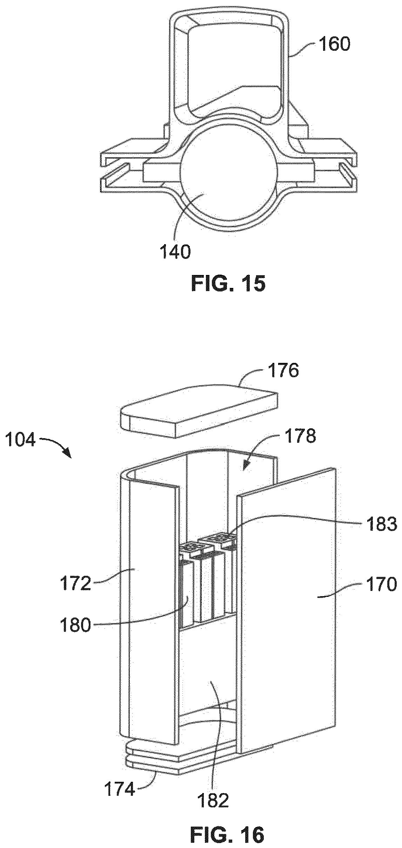

Figure 15 illustrates a perspective end view of the UV lamp 140 secured to a mounting bracket or clamp 160, according to an example of the present disclosure. Each end of the UV lamp 140 may be coupled to mounting bracket or clamp 160, which secures the UV lamp 140 to the shroud 112 (shown in Figures 12-14). A buffer, such as a thin (for example, 0.040" (0.102 cm)) sheet of silicon may be disposed between the end of the UV lamp 140 and the bracket 160. Optionally, the UV lamp 140 may be secured to the shroud 112 through brackets or clamps that differ in size and shape than shown. In other examples, the UV lamp 140 may be secured to the shroud 112 through adhesives, fasteners, and/or the like.

Figure 16 illustrates a perspective exploded view of the backpack assembly 104, according to an example of the present disclosure. The backpack assembly 104 includes a front wall 170 that couples to a rear shell 172, a base 174, and a top wall 176 (or top cap 176). An internal chamber 178 is defined between the front wall 170, the rear shell 172, the base 174, and the top wall 176. One or more batteries 180, such as rechargeable Lithium batteries, are contained within the internal chamber 178. An air generation sub-system 182 is also contained within the internal chamber 178. The air generation sub-system 182 is in fluid communication with an air tube within the hose 122 (shown in Figure 2, for example). The air generation sub-system 182 may include an airflow device, such as a vacuum generator, an air blower, and/or the like. The airflow device is configured to generate airflow to cool the UV lamp, draw air from the sanitizing head 106 into the backpack assembly 104 and out through an exhaust, draw or otherwise remove generated ozone away from the shroud 112, and/or the like.

One or more air filters 183, such as carbon filters, are within the backpack assembly 104. The air filters 183 are in communication with the air tube or other such delivery duct or line that routes air through the hose 122 and into the backpack assembly 104. The air filters 183 are configured to filter the air that is drawn into the backpack assembly 104 from the shroud 112. For example, the air filters 183 may be configured to remove, deactivate, or otherwise neutralize ozone.

The batteries 180 and/or a power supply within the backpack assembly 104 provides operating power for the UV lamp 140 of the sanitizing head 106 (shown in figure 2, for example). The top wall 176 may be removably coupled to the front wall 170 and the rear shell 172. The top wall 176 may be removed to provide access to the batteries 180 (such as to remove and/or recharge the batteries), for example. Additional space may be provided within the backpack assembly 104 for storage of supplies, additional batteries, additional components, and/or the like. In at least one example, the front wall 170, the rear shell 172, the base 174, and the top wall 176 may be formed of fiberglass epoxy.

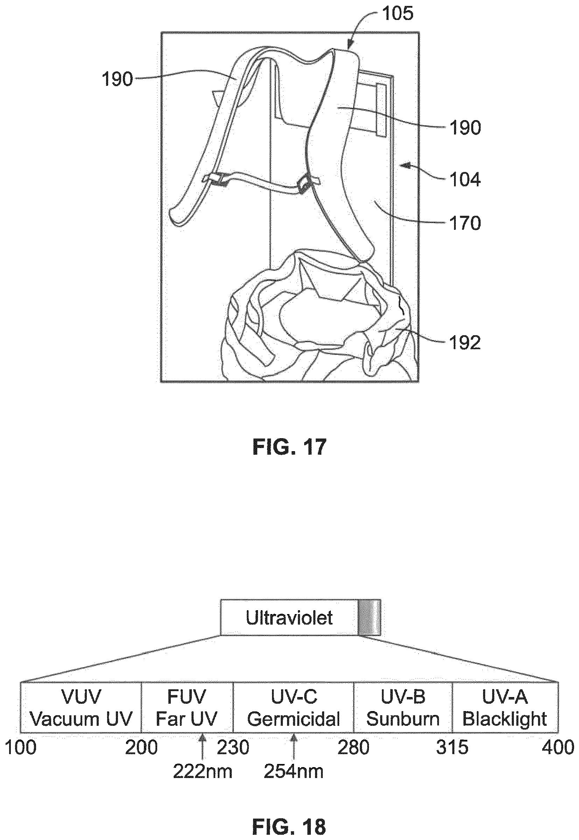

Figure 17 illustrates a perspective front view of the harness 105 coupled to the backpack assembly 104, according to an example of the present disclosure. The harness 105 may include shoulder straps 190 and/or a waist or hip belt or strap 192, which allow the individual 101 to comfortably wear the backpack assembly 104.

Referring to Figures 1-17, in operation, the individual 101 may walk through an area wearing the backpack assembly 104. When a structure to be sanitized is found, the individual 101 may grasp the handle 108 and position the sanitizing head 106 as desired, such as by extending and/or rotating the sanitizing head 106 relative to the handle 108. The individual 101 may then engage an activation button on the handle 108, for example, to activate the UV lamp 140 to emit sanitizing UV light onto the structure. As the UV lamp 140 is activated, air 150 is drawn into the shroud 112 to cool the UV lamp 140, and divert any generated ozone into the backpack assembly 104, where it is filtered by the air filters 183.

The extendable wand assembly 102 allows the sanitizing head 106 to reach distant areas, such as over an entire set of three passenger seats, from a row within an internal cabin of a commercial aircraft.

Figure 18 illustrates an ultraviolet light spectrum. Referring to Figures 1-18, in at least one example, the sanitizing head 106 is configured to emit sanitizing UV light (through operation of the UV lamp 140) within a far UV spectrum, such as between 200 nm to 230 nm. In at least one example, the sanitizing head 106 emits sanitizing UV light having a wavelength of 222 nm. In at least one other example, the sanitizing head 106 is configured to emit sanitizing UV light within a UVC spectrum, such as between 230 nm to 280 nm.

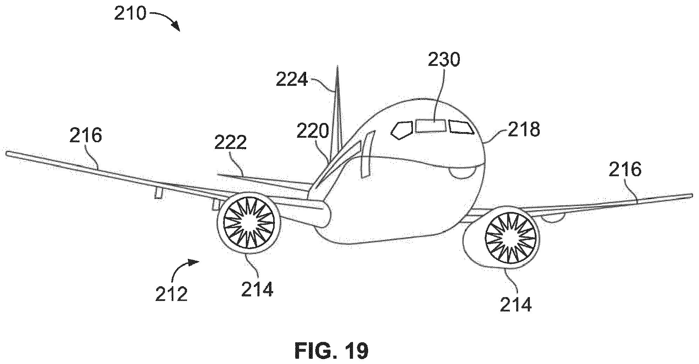

Figure 19 illustrates a perspective front view of an aircraft 210, according to an example of the present disclosure. The aircraft 210 includes a propulsion system 212 that includes engines 214, for example. Optionally, the propulsion system 212 may include more engines 214 than shown. The engines 214 are carried by wings 216 of the aircraft 210. In other examples, the engines 214 may be carried by a fuselage 218 and/or an empennage 220. The empennage 220 may also support horizontal stabilizers 222 and a vertical stabilizer 224.

The fuselage 218 of the aircraft 210 defines an internal cabin 230, which includes a flight deck or cockpit, one or more work sections (for example, galleys, personnel carry-on baggage areas, and the like), one or more passenger sections (for example, first class, business class, and coach sections), one or more lavatories, and/or the like. The internal cabin 230 includes one or more lavatory systems, lavatory units, or lavatories, as described herein.

Alternatively, instead of an aircraft, examples of the present disclosure may be used with various other vehicles, such as automobiles, buses, locomotives and train cars, watercraft, and the like. Further, examples of the present disclosure may be used with respect to fixed structures, such as commercial and residential buildings.

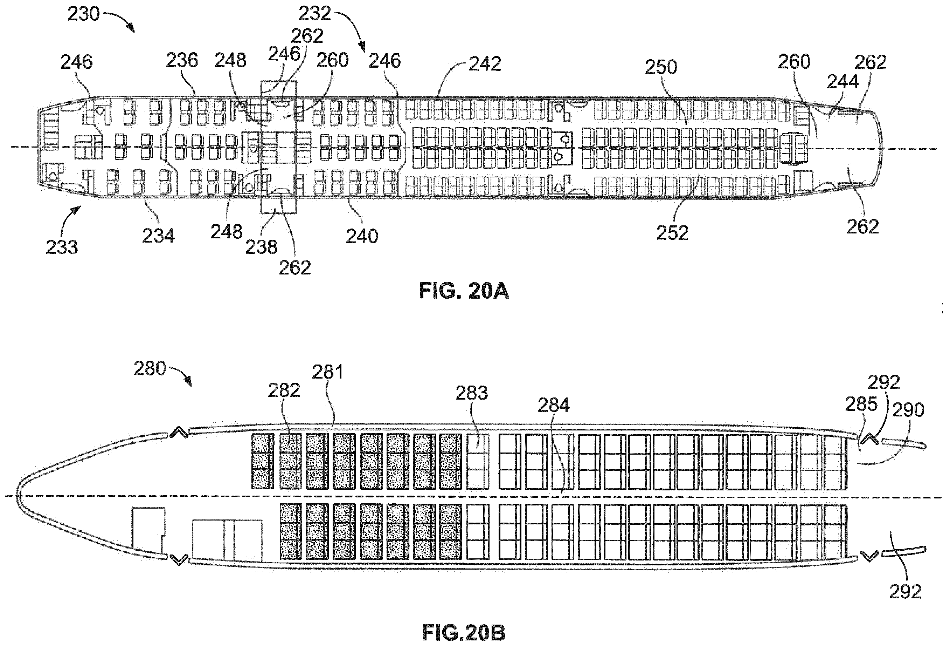

Figure 20A illustrates a top plan view of an internal cabin 230 of an aircraft, according to an example of the present disclosure. The internal cabin 230 may be within the fuselage 232 of the aircraft, such as the fuselage 218 of Figure 19. For example, one or more fuselage walls may define the internal cabin 230. The internal cabin 230 includes multiple sections, including a front section 233, a first class section 234, a business class section 236, a front galley station 238, an expanded economy or coach section 240, a standard economy of coach section 242, and an aft section 244, which may include multiple lavatories and galley stations. It is to be understood that the internal cabin 230 may include more or less sections than shown. For example, the internal cabin 230 may not include a first class section, and may include more or less galley stations than shown. Each of the sections may be separated by a cabin transition area 246, which may include class divider assemblies between aisles 248.

As shown in Figure 20A, the internal cabin 230 includes two aisles 250 and 252 that lead to the aft section 244. Optionally, the internal cabin 230 may have less or more aisles than shown. For example, the internal cabin 230 may include a single aisle that extends through the center of the internal cabin 230 that leads to the aft section 244.

The aisles 248, 250, and 252 extend to egress paths or door passageways 260. Exit doors 262 are located at ends of the egress paths 260. The egress paths 260 may be perpendicular to the aisles 248, 250, and 252. The internal cabin 230 may include more egress paths 260 at different locations than shown. The portable sanitizing system 100 shown and described with respect to Figures 1-18 may be used to sanitize various structures within the internal cabin 230, such as passenger seats, monuments, stowage bin assemblies, components on and within lavatories, galley equipment and components, and/or the like.

Figure 20B illustrates a top plan view of an internal cabin 280 of an aircraft, according to an example of the present disclosure. The internal cabin 280 is an example of the internal cabin 230 shown in Figure 19. The internal cabin 280 may be within a fuselage 281 of the aircraft. For example, one or more fuselage walls may define the internal cabin 280. The internal cabin 280 includes multiple sections, including a main cabin 282 having passenger seats 283, and an aft section 285 behind the main cabin 282. It is to be understood that the internal cabin 280 may include more or less sections than shown.

The internal cabin 280 may include a single aisle 284 that leads to the aft section 285. The single aisle 284 may extend through the center of the internal cabin 280 that leads to the aft section 285. For example, the single aisle 284 may be coaxially aligned with a central longitudinal plane of the internal cabin 280.

The aisle 284 extends to an egress path or door passageway 290. Exit doors 292 are located at ends of the egress path 290. The egress path 290 may be perpendicular to the aisle 284. The internal cabin 280 may include more egress paths than shown. The portable sanitizing system 100 shown and described with respect to Figures 1-18 may be used to sanitize various structures within the internal cabin 230, such as passenger seats, monuments, stowage bin assemblies, components on and within lavatories, galley equipment and components, and/or the like.

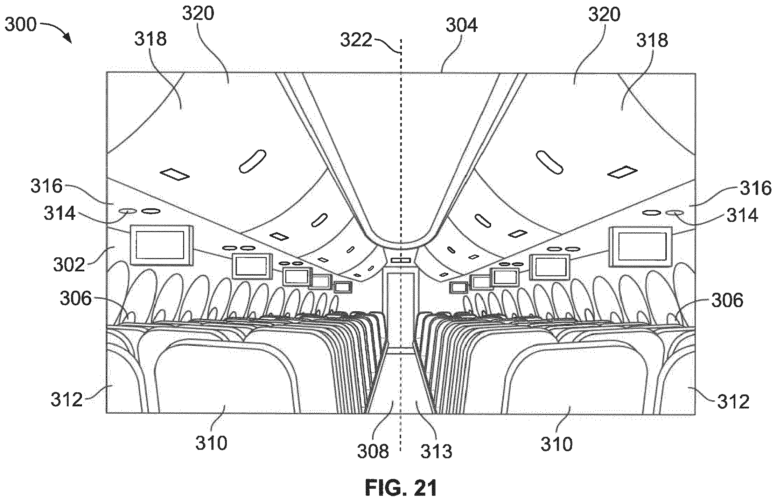

Figure 21 illustrates a perspective interior view of an internal cabin 300 of an aircraft, according to an example of the present disclosure. The internal cabin 300 includes outboard walls 302 connected to a ceiling 304. Windows 306 may be formed within the outboard walls 302. A floor 308 supports rows of seats 310. As shown in Figure 21, a row 312 may include two seats 310 on either side of an aisle 313. However, the row 312 may include more or less seats 310 than shown. Additionally, the internal cabin 300 may include more aisles than shown.

Passenger service units (PSUs) 314 are secured between an outboard wall 302 and the ceiling 304 on either side of the aisle 313. The PSUs 314 extend between a front end and rear end of the internal cabin 300. For example, a PSU 314 may be positioned over each seat 310 within a row 312. Each PSU 314 may include a housing 316 that generally contains vents, reading lights, an oxygen bag drop panel, an attendant request button, and other such controls over each seat 310 (or groups of seats) within a row 312.

Overhead stowage bin assemblies 318 are secured to the ceiling 304 and/or the outboard wall 302 above and inboard from the PSU 314 on either side of the aisle 313. The overhead stowage bin assemblies 318 are secured over the seats 310. The overhead stowage bin assemblies 318 extend between the front and rear end of the internal cabin 300. Each stowage bin assembly 318 may include a pivot bin or bucket 320 pivotally secured to a strongback (hidden from view in Figure 21). The overhead stowage bin assemblies 318 may be positioned above and inboard from lower surfaces of the PSUs 314. The overhead stowage bin assemblies 318 are configured to be pivoted open in order to receive passenger carry-on baggage and personal items, for example.

As used herein, the term "outboard" means a position that is further away from a central longitudinal plane 322 of the internal cabin 300 as compared to another component. The term "inboard" means a position that is closer to the central longitudinal plane 322 of the internal cabin 300 as compared to another component. For example, a lower surface of a PSU 314 may be outboard in relation to a stowage bin assembly 318.

The portable sanitizing system 100 shown and described with respect to Figures 1-18 may be used to sanitize various structures shown within the internal cabin 300. As an example, the portable sanitizing system 100 may be used to sanitize various components within a cockpit or flight deck of an aircraft.

When not in use, the portable sanitizing system 100 may be stored within a closet, galley cart bay, or galley cart, such as within the internal cabin of the vehicle.

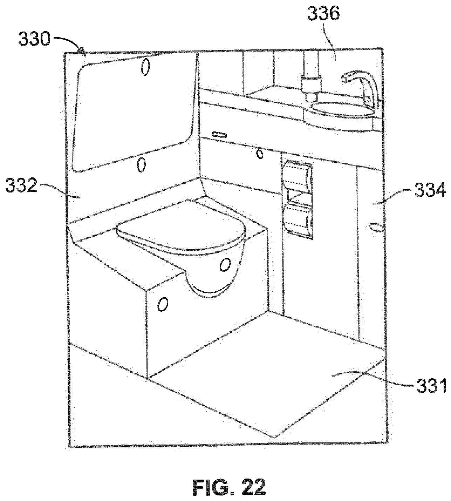

Figure 22 illustrates a perspective internal view of a lavatory 330 within an internal cabin of a vehicle, such as any of the internal cabins described herein. The lavatory 330 is an example of an enclosed space, monument or chamber, such as within the internal cabin a vehicle. The lavatory 330 may be onboard an aircraft, as described above. Optionally, the lavatory 330 may be onboard various other vehicles. In other examples, the lavatory 330 may be within a fixed structure, such as a commercial or residential building. The lavatory 330 includes a base floor 331 that supports a toilet 332, cabinets 334, and a sink 336 or wash basin. The lavatory 330 may be arranged differently than shown. The lavatory 330 may include more or less components than shown. The portable sanitizing system 100 shown and described with respect to Figures 1-18 may be used to sanitize the various structures, components, and surfaces within the lavatory 330.

Certain examples of the present disclosure provide a portable sanitizing method including coupling a backpack assembly to a wand assembly including an ultraviolet (UV) lamp. In at least one example, said coupling includes coupling the backpack assembly to the wand assembly with a hose.

In at least one example, the portable sanitizing method further includes generating airflow to cool the UV lamp by an airflow device of the backpack assembly; drawing air from a sanitizing head of the wand assembly by the airflow device of the backpack assembly; and/or removing ozone from the sanitizing head of the wand assembly by the airflow device of the backpack assembly.

In at least one example, the portable sanitizing method includes filtering air drawn from a sanitizing head of the wand assembly by at least one air filter of the backpack assembly.

In at least one example, the portable sanitizing method further includes providing power to the UV lamp by one or more batteries contained within the backpack assembly.

Referring to Figures 1-22, the portable sanitizing system 100 can be used to safely and effectively sanitize high-touch surfaces in the flight deck and internal cabin in a timely and cost-effective manner. UV disinfection allows the internal cabin to be quickly and effectively disinfected, such as between flights. In at least one example, the portable sanitizing system 100 is used to augment a cleaning process, such as after manual cleaning.

As described herein, examples of the present disclosure provide systems and a methods for efficiently sterilizing surfaces, components, structures, and/or the like within an internal cabin of a vehicle. Further, examples of the present disclosure provide compact, easy-to-use, and safe systems and methods for using UV light to sterilize surfaces within an internal cabin.

While various spatial and directional terms, such as top, bottom, lower, mid, lateral, horizontal, vertical, front and the like can be used to describe examples of the present disclosure, it is understood that such terms are merely used with respect to the orientations shown in the drawings. The orientations can be inverted, rotated, or otherwise changed, such that an upper portion is a lower portion, and vice versa, horizontal becomes vertical, and the like.

As used herein, a structure, limitation, or element that is "configured to" perform a task or operation is particularly structurally formed, constructed, or adapted in a manner corresponding to the task or operation. For purposes of clarity and the avoidance of doubt, an object that is merely capable of being modified to perform the task or operation is not "configured to" perform the task or operation as used herein.

It is to be understood that the above description is intended to be illustrative, and not restrictive. For example, the above-described examples (and/or features thereof) can be used in combination with each other. In addition, many modifications can be made to adapt a particular situation or material to the teachings of the various examples of the disclosure without departing from the scope of the invention defined in the appended claims. While the dimensions and types of materials described herein are intended to define the parameters of the various examples of the disclosure, the examples are by no means limiting. Many other examples will be apparent to those of skill in the art upon reviewing the above description. The scope of the various examples of the disclosure should, therefore, be determined with reference to the appended claims. In the appended claims and the detailed description herein, the terms "including" and "in which" are used as the plain-English equivalents of the respective terms "comprising" and "wherein." Moreover, the terms "first," "second," and "third," etc. are used merely as labels, and are not intended to impose numerical requirements on their objects.

This written description uses examples to disclose the various examples of the disclosure, including the best mode, and also to enable any person skilled in the art to practice the various examples of the disclosure, including making and using any devices or systems and performing any incorporated methods. The patentable scope of the various examples of the disclosure is defined by the claims, and can include other examples that occur to those skilled in the art.

## Claims

1. A portable sanitizing system (100), comprising:
a wand assembly (102) including a sanitizing head (106) having an ultraviolet (UV) lamp (140); and
a backpack assembly (104) coupled to the wand assembly (102), wherein the backpack assembly (104) is removably securable to an individual (101) through a harness (105);
wherein the backpack assembly (104) comprises an airflow device that is configured to generate airflow to cool the UV lamp (140), and to draw air (150) from the sanitizing head (106) of the wand assembly (102).

2. The portable sanitizing system (100) of claim 1, further comprising a hose (122) that couples the backpack assembly (104) to the wand assembly (102).

3. The portable sanitizing system (100) of claim 1 or 2, wherein the airflow device is further configured to remove ozone from the sanitizing head (106).

4. The portable sanitizing system (100) of any one of claims 1-3, wherein the backpack assembly (104) comprises at least one air filter (183) that is configured to filter air (150) drawn from the sanitizing head (106) of the wand assembly (102).

5. The portable sanitizing system (100) of any one of claims 1-4, wherein the backpack assembly (104) contains one or more batteries (180) that provide power to the UV lamp (140).

6. The portable sanitizing system (100) of any one of claims 1-5, wherein the UV lamp (140) is configured:
to emit the UV light having a wavelength between 200 nm - 230 nm, optionally 222 nm; or
to emit the UV light having a wavelength between 230 nm - 280 nm, optionally 254 nm.

7. The portable sanitizing system (100) of any preceding claim, wherein the wand assembly (102) in a stowed position is removably secured to a portion of the backpack assembly (104) through one or more tracks, clips, latches, belts, or ties.

8. The portable sanitizing system (100) of any preceding claim, wherein the wand assembly (102) further comprises a handle (108) coupled to the sanitizing head (106) and, optionally, a coupler (110) that moveably couples the handle (108) to the sanitizing head (106).

9. The portable sanitizing system (100) of claim 8, wherein the sanitizing head (106) is configured to linearly translate and/or swivel in relation to the handle (108).

10. The portable sanitizing system (100) of any preceding claim, wherein the sanitizing head (106) comprises a shroud (112) that retains the UV lamp (140) and, optionally, the shroud (112) comprises one or more openings (152) that are configured to allow air (150) to flow into the shroud (112).

11. The portable sanitizing system (100) of claim 10, further comprising a reflector (142) secured to an underside of the shroud (112), wherein the reflector (142) is configured to reflect a portion of the UV light that is emitted by the UV lamp (140).

12. The portable sanitizing system (100) of any preceding claim, wherein the sanitizing head (106) further comprises:
a reflector (142), optionally the reflector (142) of claim 11; and
a cover plate (154),
wherein the UV lamp (140) is secured within an interior chamber (156) defined between the reflector (142) and the cover plate (154).

13. A method of sanitizing a structure of a vehicle, such as a cockpit and/or a passenger section of an internal cabin (230) of a commercial aircraft (210), using the portable sanitizing system (100) of any one of claims 1-12, the method comprising:
an individual (101) wearing the portable sanitizing system (100) while the walking through the vehicle as the UV lamp (140) emits the UV light onto the structure.

14. The method of claim 13 when dependent upon claim 9, comprising:
the individual (101) grasping the handle (108) and positioning the sanitizing head (106) by linearly translating and/or swivelling the sanitizing head (106) relative to the handle (108).

15. The method of claim 13, when dependent upon claims 4 and 10, or claim 14 when dependent upon claim 4, comprising:
the individual (101) engaging an activation button on the handle (108) thereby activating the UV lamp (140) to emit sanitizing UV light onto the structure and causing air (150) to be drawn into the shroud (112) to cool the UV lamp (140) and any generated ozone to be diverted into the backpack assembly (104) where it is filtered by the at least one air filter (183).

## Patentansprüche

1. Tragbares Desinfektionssystem (100), umfassend:
eine Stabbaugruppe (102), die einen Desinfektionskopf (106) mit einer Ultraviolett-(UV)-Lampe (140) enthält; und
eine Rucksackbaugruppe (104), die mit der Stabbaugruppe (102) verbunden ist, wobei die Rucksackbaugruppe (104) durch ein Gurtzeug (105) abnehmbar an einer Person (101) befestigbar ist;
wobei die Rucksackbaugruppe (104) eine Luftstromvorrichtung umfasst, die konfiguriert ist, um einen Luftstrom zum Kühlen der UV-Lampe (140) zu erzeugen und um Luft (150) aus dem Desinfektionskopf (106) der Stabbaugruppe (102) abzusaugen.

2. Tragbares Desinfektionssystem (100) nach Anspruch 1, das ferner einen Schlauch (122) umfasst, der die Rucksackanordnung (104) mit der Stabanordnung (102) verbindet.

3. Tragbares Desinfektionssystem (100) nach Anspruch 1 oder 2, bei dem die Luftstromvorrichtung ferner konfiguriert ist, um Ozon aus dem Desinfektionskopf (106) zu beseitigen.

4. Tragbares Desinfektionssystem (100) nach einem der Ansprüche 1 bis 3, bei dem die Rucksackbaugruppe (104) mindestens einen Luftfilter (183) umfasst, der zum Filtern der vom Desinfektionskopf (106) der Stabbaugruppe (102) abgesaugten Luft (150) konfiguriert ist.

5. Tragbares Desinfektionssystem (100) nach einem der Ansprüche 1-4, bei dem die Rucksackbaugruppe (104) eine oder mehrere Batterien (180) enthält, die die UV-Lampe (140) mit Strom versorgen.

6. Tragbares Desinfektionssystem (100) nach einem der Ansprüche 1-5, bei dem die UV-Lampe (140) konfiguriert ist zum:
Emittieren des UV-Lichts mit einer Wellenlänge zwischen 200 nm - 230 nm, optional 222 nm; oder
Emittieren des UV-Lichts mit einer Wellenlänge zwischen 230 nm und 280 nm, optional 254 nm.

7. Tragbares Desinfektionssystem (100) nach einem der vorhergehenden Ansprüche, bei dem die Stabbaugruppe (102) in einer verstauten Position abnehmbar an einem Teil der Rucksackbaugruppe (104) durch Schienen, Klammern, Verriegelungen, Gurte und/oder Bänder befestigt ist.

8. Tragbares Desinfektionssystem (100) nach einem der vorhergehenden Ansprüche, bei dem die Stabbaugruppe (102) ferner einen mit dem Desinfektionskopf (106) gekoppelten Griff (108) umfasst, und,
optional ein Verbindungsstück (110) umfasst, das den Griff (108) beweglich mit dem Reinigungskopf (106) verbindet.

9. Tragbares Desinfektionssystem (100) nach Anspruch 8, bei dem der Desinfektionskopf (106) konfiguriert ist, um sich in Bezug auf den Griff (108) linear zu verschieben und/oder zu schwenken.

10. Tragbares Desinfektionssystem (100) nach einem der vorhergehenden Ansprüche, bei dem der Desinfektionskopf (106) eine Abdeckung (112) umfasst, die die UV-Lampe (140) aufnimmt, und die Abdeckung (112) optional eine oder mehrere Öffnungen (152) umfasst, die konfiguriert sind, um Luft (150) in die Abdeckung (112) strömen zu lassen.

11. Tragbares Desinfektionssystem (100) nach Anspruch 10, das ferner einen Reflektor (142) umfasst, der an einer Unterseite der Abdeckung (112) befestigt ist, wobei der Reflektor (142) konfiguriert ist, um einen Teil des von der UV-Lampe (140) emittierten UV-Lichts zu reflektieren

12. Tragbares Desinfektionssystem (100) nach einem der vorhergehenden Ansprüche, bei dem der Desinfektionskopf (106) ferner umfasst:
einen Reflektor (142), optional den Reflektor (142) nach Anspruch 11; und
eine Abdeckplatte (154),
wobei die UV-Lampe (140) in einer Innenkammer (156) befestigt ist, die zwischen dem Reflektor (142) und der Abdeckplatte (154) definiert ist.

13. Verfahren zum Desinfizieren einer Struktur eines Fahrzeugs, wie eines Cockpits und/oder eines Passagierbereichs einer Innenkabine (230) eines Verkehrsflugzeugs (210), unter Verwendung des tragbaren Desinfektionssystems (100) nach einem der Ansprüche 1 bis 12, wobei das Verfahren umfasst, dass
eine Person (101), die das tragbare Desinfektionssystem (100) trägt, durch das Fahrzeug geht, und dabei die UV-Lampe (140) das UV-Licht auf die Struktur abstrahlt.

14. Verfahren nach Anspruch 13 in Abhängigkeit von Anspruch 9, umfassend, dass:
die Person (101) den Griff (108) fasst und den Desinfektionskopf (106) durch lineares Verschieben und/oder Schwenken des Desinfektionskopfes (106) relativ zum Griff (108) positioniert.

15. Verfahren nach Anspruch 13, soweit von den Ansprüchen 4 und 10 abhängig, oder nach Anspruch 14, soweit von Anspruch 4 abhängig, umfassend, dass:
die Person (101) einen Aktivierungsknopf am Griff (108) betätigt, und dadurch die UV-Lampe (140) aktiviert, um desinfizierendes UV-Licht auf die Struktur zu emittieren, und bewirkt, dass Luft (150) in die Abdeckung (112) gesaugt wird, um die UV-Lampe (140) zu kühlen, und dass jegliches erzeugte Ozon in die Rucksackbaugruppe (104) abgeleitet wird, wo es von dem mindestens einen Luftfilter (183) gefiltert wird.

## Revendications

1. Système de désinfection portable (100), comprenant :
un ensemble lance (102) comportant une tête de désinfection (106) ayant une lampe à ultraviolet (UV) (140) ; et
un ensemble sac à dos (104) couplé à l'ensemble lance (102), dans lequel l'ensemble sac à dos (104) peut être fixé de manière amovible à un individu (101) par l'intermédiaire d'un harnais (105) ;
dans lequel l'ensemble sac à dos (104) comprend un dispositif de circulation d'air qui est configuré pour générer un flux d'air pour refroidir la lampe UV (140), et pour aspirer de l'air (150) depuis la tête de désinfection (106) de l'ensemble lance (102).

2. Système de désinfection portable (100) selon la revendication 1, comprenant en outre un tuyau (122) qui relie l'ensemble sac à dos (104) à l'ensemble lance (102).

3. Système de désinfection portable (100) selon la revendication 1 ou 2, dans lequel le dispositif de circulation d'air est en outre configuré pour éliminer de l'ozone depuis la tête de désinfection (106).

4. Système de désinfection portable (100) selon l'une quelconque des revendications 1 à 3, dans lequel l'ensemble sac à dos (104) comprend au moins un filtre à air (183) qui est configuré pour filtrer de l'air (150) aspiré depuis la tête de désinfection (106) de l'ensemble lance (102).

5. Système de désinfection portable (100) selon l'une quelconque des revendications 1 à 4, dans lequel l'ensemble sac à dos (104) contient une ou plusieurs batteries (180) qui alimentent en énergie la lampe UV (140).

6. Système de désinfection portable (100) selon l'une quelconque des revendications 1 à 5, dans lequel la lampe UV (140) est configurée :
pour émettre la lumière UV ayant une longueur d'onde comprise entre 200 nm et 230 nm, facultativement 222 nm ; ou
pour émettre la lumière UV ayant une longueur d'onde comprise entre 230 nm et 280 nm, facultativement 254 nm.

7. Système de désinfection portable (100) selon l'une quelconque des revendications précédentes, dans lequel l'ensemble lance (102) en position rangée est fixé de manière amovible à une partie de l'ensemble sac à dos (104) par l'intermédiaire d'un ou plusieurs rails, clips, cliquets, courroies, ou liens.

8. Système de désinfection portable (100) selon l'une quelconque des revendications précédentes, dans lequel l'ensemble lance (102) comprend en outre une poignée (108) couplée à la tête de désinfection (106) et, facultativement, un coupleur (110) qui couple de manière mobile la poignée (108) à la tête de désinfection (106).

9. Système de désinfection portable (100) selon la revendication 8, dans lequel la tête de désinfection (106) est configurée pour se déplacer linéairement et/ou pivoter par rapport à la poignée (108).

10. Système de désinfection portable (100) selon l'une quelconque des revendications précédentes, dans lequel la tête de désinfection (106) comprend un capot (112) qui retient la lampe UV (140) et, facultativement, le capot (112) comprend une ou plusieurs ouvertures (152) qui sont configurées pour permettre à de l'air (150) de circuler dans le capot (112).

11. Système de désinfection portable (100) selon la revendication 10, comprenant en outre un réflecteur (142) fixé à un côté inférieur du capot (112), dans lequel le réflecteur (142) est configuré pour réfléchir une partie de la lumière UV qui est émise par la lampe UV (140).

12. Système de désinfection portable (100) selon l'une quelconque des revendications précédentes, dans lequel la tête de désinfection (106) comprend en outre :
un réflecteur (142), facultativement le réflecteur (142) selon la revendication 11 ; et
une plaque de recouvrement (154),
dans lequel la lampe UV (140) est fixée à l'intérieur d'une chambre intérieure (156) définie entre le réflecteur (142) et la plaque de recouvrement (154).

13. Procédé de désinfection d'une structure d'un véhicule, telle qu'un cockpit et/ou une section passagers d'une cabine interne (230) d'un aéronef commercial (210), utilisant le système de désinfection portable (100) selon l'une quelconque des revendications 1 à 12, le procédé comprenant l'étape suivante :
un individu (101) porte le système de désinfection portable (100) tout en se déplaçant dans le véhicule, la lampe UV (140) émettant ainsi la lumière UV sur la structure.

14. Procédé selon la revendication 13 lorsqu'elle dépend de la revendication 9, comprenant l'étape suivante :
l'individu (101) saisit la poignée (108) et positionne la tête de désinfection (106) en déplaçant linéairement et/ou en faisant pivoter la tête de désinfection (106) par rapport à la poignée (108).

15. Procédé selon la revendication 13, lorsqu'elle dépend des revendications 4 et 10, ou selon la revendication 14 lorsqu'elle dépend de la revendication 4, comprenant l'étape suivante :
l'individu (101) actionne un bouton d'activation sur la poignée (108), activant ainsi la lampe UV (140) pour émettre une lumière UV de désinfection sur la structure et amenant de l'air (150) à être aspiré dans le capot (112) pour refroidir la lampe UV (140) et amenant tout ozone généré à être redirigé dans l'ensemble sac à dos (104) où il est filtré par le au moins un filtre à air (183).
